(19) Europäisches Patentamt
European Patent Office
Office européen des brevets

(11) Numéro de publication: **0 151 072 B1**

# (12) FASCICULE DE BREVET EUROPEEN

(45) Date de publication du fascicule du brevet: **09.03.88**

(51) Int. Cl.[4]: **C 07 F 9/38**, C 07 F 9/58, C 07 F 9/65, A 61 K 31/66

(21) Numéro de dépôt: **85400113.8**

(22) Date de dépôt: **24.01.85**

## (54) Dérivés de l'acide méthylènediphosphonique, procédé d'obtention et compositions pharmaceutiques antirhumatismales les contenant.

(30) Priorité: **26.01.84 FR 8401214**

(43) Date de publication de la demande: **07.08.85 Bulletin 85/32**

(45) Mention de la délivrance du brevet: **09.03.88 Bulletin 88/10**

(84) Etats contractants désignés: **AT BE CH DE FR GB IT LI LU NL SE**

(56) Documents cités:
**EP - A - 0 015 370**
**EP - A - 0 098 567**
**DE - A - 2 754 821**

(73) Titulaire: **SANOFI, 40, Avenue George V, F-75008 Paris (FR)**

(72) Inventeur: **Barbier, Alain, 280, Avenue du Miradou, F-34980 St-Clement La Riviere (FR)**
Inventeur: **Breliere, Jean-Claude, 1065, rue de l'Aiguelongue Villa 112, F-34100 Montpellier (FR)**
Inventeur: **Garcia, Georges, 27, rue des Aires, F-34980 St-Gely-du-Fesc (FR)**

(74) Mandataire: **Gillard, Marie-Louise et al, Cabinet Beau de Loménie 55, Rue d'Amsterdam, F-75008 Paris (FR)**

**EP 0 151 072 B1**

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

## Description

La présente invention a pour objet de nouveaux dérivés de l'acide méthylènediphosphonique doués de propriétés thérapeutiques permettant leur utilisation dans le traitement des manifestations rhumatismales.

Plus précisément, les composés selon l'invention répondent à la formule générale:

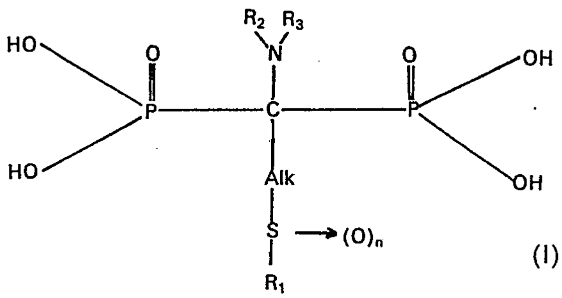

(I)

dans laquelle:

$R_1$ représente:

– un groupe alkyle en $C_1$–$C_6$

– un groupe cycloalkyle en $C_5$–$C_7$

– un groupe phényle éventuellement substitué une ou plusieurs fois par un halogène, un groupe alkyle en $C_1$–$C_6$, un groupe trifluorométhyle,

– un hétérocycle à 5 ou 6 chaînons comportant 1 ou 2 hétéroatomes choisis parmi l'azote et le soufre,

Alk désigne un groupe alkylène droit ou ramifié en $C_1$–$C_6$,

$R_2$ représente l'hydrogène, un groupe alkyle en $C_1$–$C_6$ ou un groupe $-CONH_2$

$R_3$ représente l'hydrogène, un groupe alkyle en $C_1$–$C_6$, un groupe benzyle ou un groupe phényle éventuellement substitué par un chlore ou un méthyle;

ou encore $R_2$ et $R_3$ pris ensemble représentent un groupe $(CH_2)_m$ où m=4 ou 5 enfin, <u>n</u> représente un nombre entier 0, 1 ou 2.

Les acides de formule (I) sont susceptibles de fournir des sels avec les bases minérales ou organiques. Ces sels font partie intégrante de l'invention. L'un des composés de formule (I) a déjà été décrit, mais en aucun cas ses propriétés thérapeutiques n'ont été mentionnées.

Ainsi, le brevet en Allemagne Fédérale n° 2 754 821 décrit le composé de formule (I) où $R_1=CH_3$; n=0; Alk est $-CH_2-CH_2-$; $R_2=R_3=H$.

Ce brevet revendique également les sels alcalins et alcalinoterreux correspondants, ainsi que l'utilisation des produits comme additifs dans le traitement des eaux dans l'industrie textile et papetière.

Les autres composés (I) sont nouveaux.

La présente invention comporte également un procédé d'obtention des composés de formule (I). Dans le cas des composés (I) où $R_2=R_3=H$, on utilise comme produit de départ un nitrile $R_1S(O)_n$–Alk–CN où $R_1$, Alk et n ont la signification précédemment définie. Ces composés sont préparés selon des méthodes connues soit par action d'un cyanure alcalin sur un alkylmercaptan ω bromé ($R_1S(O)_n$–Alk–Br), soit par action d'un nitrile ω halogéné sur un thiol en présence d'une base minérale.

Dans le cas où n=0, et Alk est $-CH_2-CH_2-$, une variante du procédé consiste à faire réagir le thiol RISH sur l'acrylonitrile en présence d'une base organique selon le procédé décrit par C.D. HURD et L.L. GERSHBEIN (J. Amer. Chem. Soc. 69 2328 1947).

Le nitrile ainsi préparé est ensuite chauffé en présence d'acide phosphoreux à une température comprise entre 140 °C et 200 °C pendant une période allant de 1 heure à quelques heures.

Une variante de ce procédé consiste à ajouter le nitrile à une solution de tribromure de phosphore dans un solvant convenable, tel que le dioxanne en présence de la quantité stoechiométrique d'eau. La température du milieu peut varier selon les cas entre 20 °C et 70 °C. Le mélange est ensuite agité pendant un temps qui varie de quelques heures à 1 journée à une température variant entre 30 °C et 70 °C.

Quelle que soit la signification des substituants $R_2$ et $R_3$, on peut aussi préparer les composés (I) à partir des amides de $R_1-S(O)_n-Alk-C(=O)-N(R_2)(R_3)$ comme produit de départ, dans lesquels $R_1$, n, Alk, $R_2$ et $R_3$ sont tels que définis ci-dessus.

Ces amides s'obtiennent de façon connue à partir des acides $R_1-S(O)_n-Alk-COOH$ par exemple par action de chlorure de thionyle ou de trichlorure de phosphore sur l'acide suivie de réaction avec l'amine $HN(R_2)(R_3)$.

L'amide ainsi préparé est ensuite chauffé avec de l'acide phosphoreux et du trichlorure de phosphore au sein d'un solvant inerte comme le diméthoxyéthane à une température comprise entre 50 et 100 °C pendant 2 à 6 heures.

Les acides phosphoriques ainsi obtenus peuvent être transformés de façon connue en l'un de leurs sels. L'opération est effectuée dans un solvant chaud de façon que le sel cristallise par refroidissement après addition éventuelle d'un tiers solvant.

Les exemples suivants sont donnés à titre d'illustration de l'invention.

Exemple 1

Acide amino-1 (méthyl-4 phényl thio)-2 éthylidène diphosphonique-1,1 sel tétrasodique, dihydraté (SR 42710).

$R_1$ = 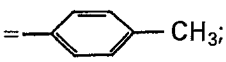 ; n=0; Alk = $-CH_2-$;

$R_2=R_3=H$ I

a) Méthyl 4-phényl thio acétonitrile préparé selon la méthode décrite par A.A. SANTILLI (C.A. 75 P 5935 S).

b) Acide amino 1-(méthyl-4 phényl thio)-2-éthylidène diphosphonique-1,1.

A une solution de 133 g de tribromure de phosphore dans 150 ml de dioxanne, on ajoute, à 30 °C, 40 g de méthyl-4 phényl-thioacétonitrile et on laisse réagir sous agitation pendant 30 min. On additionne ensuite goutte à goutte 27 g d'eau en refroidissant le milieu réactionnel de telle sorte que la température n'excède pas 70 °C. Après 3 h de réaction à 70 °C, on concentre partiellement le dioxanne et on précipite le produit par addition d'eau. Après filtration du précipité, lavage à l'eau puis à l'acétone, ce dernier est séché.

Une solution de 5 g du produit précédent et de 2,5 g d'hydroxyde de sodium dans 100 ml d'eau distillée est chauffée à reflux pendant 5 min. La solution est filtrée à chaud puis refroidie à 50 °C; on ajoute alors 100 ml de méthanol et on laisse refroidir le mélange. Le précipité est filtré, lavé au méthanol puis séché à 80 °C sous vide. On obtient ainsi 4,8 g de tétra sel de sodium; de l'acide amino-1 (méthyl-4 phényl thio)-2 éthylidène diphosphonique-1,1; F > 300 °C.

Analyse élémentaire avec 2 molécules d'eau

| | | | | |
|---|---|---|---|---|
| Calculé | C% 23,95 | H% 3,35 | N% 3,10 | S% 7,10 |
| Trouvé | 23,67 | 3,28 | 3,06 | 6,83 |

Exemple 2

Acide amino-1 (trifluoro méthyl-3 phényl thio)-3 propylidène diphosphonique-1,1, sel tétrasodique trihydraté (SR 42707 A).

$R_1$ = 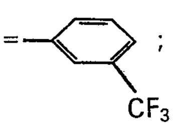 ; n=0; Alk$-(CH_2)_2-$; $R_2=R_3=H$ I

a) Trifluoro méthyl-3 phényl thio)-3 propionitrile.

48 ml d'acrylonitrile sont ajoutés goutte à goutte à une solution de 61 g de trifluoro méthyl-3 thiophénol et 2 ml de pipéridine; la température du milieu réactionnel étant maintenue inférieure à 20 °C. On laisse reposer le mélange 48 h puis on le reprend par 300 ml d'éther. Après lavage à l'eau puis séchage de la solution ethérée, on concentre. On obtient une huile qui est chromatographiée sur silice (230–400 Mesh). La fraction éluée par le mélange hexane-acétone (90/10) fournit le produit attendu sous forme d'une huile (21 g) caractérisée par chromatographie en couche mince de silice (éluant: éther isopropylique) par un RF de 0,6.

b) Tétra sel de sodium de l'acide amino-1 (trifluoro-méthyl-3 phényl thio)-3 propylidène diphosphonique-1,1, trihydraté.

A une solution de 25 g de tribromure de phosphore dans 40 ml de dioxanne, on ajoute goutte à goutte 10,5 g du nitrile précédent, à une température n'excédant pas 70 °C. On chauffe ensuite 3 h à 80 °C puis on refroidit et on ajoute 150 ml d'eau. L'insoluble est filtré, lavé à l'éther éthylique puis à l'acétone et enfin séché sous vide. Le précipité séché est dissous dans une solution de 500 mg de soude dans 15 ml d'eau: la solution résultante est filtrée puis on ajoute 50 ml de méthanol. Le précipité obtenu est filtré, lavé au méthanol puis à l'éther et fournit 1,5 g du sel attendu, après séchage. F > 300 °C.

Analyse élémentaire avec 3 molécules d'eau

| | | | |
|---|---|---|---|
| Calculé | C% 22,48 | H% 3,01 | N% 2,62 |
| Trouvé | 22,21 | 2,38 | 2,33 |

Exemple 3

Acide amino-1 (dichloro-3,4 phényl thio)-3-propylidène diphosphonique-1,2, monohydraté (SR 42683).

$R_1$ = 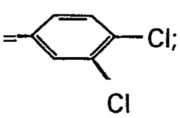 ; n=0; Alk=$(CH_2)_2$; $R_2=R_3=H$ I

a) (Dichloro-3,4 phényl)thio-3 propionitrile.

10 g de dichloro-3,4 thio phénol sont ajoutés à une solution de 0,2 ml de pipéridine dans 4 ml d'acétonitrile. On agite ensuite le mélange pendant 20 h à 20 °C puis on distille sous 0,2 mm de mercure. On recueille 12 g du produit attendu distillant entre 138 °C et 142 °C sous forme d'une huile incolore. Cette dernière est caractérisée en chromatographie en couche mince sur silice (Kieselgel 60 F 54: Merck) avec l'éther isopropylique comme éluant par un RF de 0,53.

b) Acide amino-1 (dichloro 3,4-phényl thio)-3 propoylidène diphosphonique-1,1, monohydraté.

A une solution de 10 ml de tribromure de phosphore dans 18 ml de dioxanne, on ajoute à 20 °C, en 1 min, 12 g du nitrile précédent. Le mélange est ensuite agité pendant 20 h à température ordinaire puis on ajoute 5,5 ml d'eau distillée en maintenant la température à 30 °C. On chauffe ensuite 3 h à 60 °C. Le mélange refroidi est repris par 200 ml d'acétone auquel on ajoute 10 ml d'eau.

Le précipité formé est filtré, lavé à l'acétone puis à l'éther et séché. On obtient 11,5 g du produit attendu. F: 260–261 °C (sec.)

Analyse élémentaire avec 1 molécule d'eau

| | | | | | | |
|---|---|---|---|---|---|---|
| Calculé | C% | 26,10 | H% | 3,65 | N% | 3,38 |
| Trouvé | | 25,3 | | 3,26 | | 3,58 |

Exemple 4

Sel tétrasodique de l'acide amino-1 (dichloro-3,4 phényl thio)-3 propylidène diphosphonique-1,2, monohydraté (SR 42683 A).

$R_1$ = 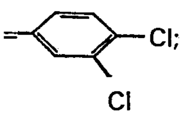 ; n=0; Alk=$(CH_2)_2$; $R_2$=$R_3$=H I

11,4 g de l'acide préparé dans l'exemple 3 (SR 42683) sont traités à 50 °C par une solution de 4,6 g de soude dans 125 ml d'eau. Après dissolution complète, on ajoute 300 ml de méthanol. Le précipité est filtré, lavé au méthanol puis à l'éther pour conduire, après séchage sous vide à 80 °C, à 12,6 g du sel tétrasodique attendu. F > 300 °C.

Analyse élémentaire avec 1 molécule d'eau

| | | | | | | |
|---|---|---|---|---|---|---|
| Calculé | C% | 21,52 | H% | 2,21 | N% | 2,79 |
| Trouvé | | 21 | | 2,41 | | 2,51 |

Exemple 5

Sel tétrasodique de l'acide amino-1 phényl thio-4 butylidène diphosphonique-1,1, trihydraté (SR 42718 A).

$R_1$= 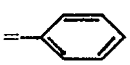 ; n=0; Alk=$(CH_2)_3$; $R_2$=$R_3$=H I

a) Phényl thio-4 butyronitrile préparé selon la méthode décrite par J.W. LYNN (C.A. 56 1141 e).

b) Sel tétrasodique de l'acide amino-1 phényl thio-4 butylidène diphosphonique-1,1, trihydraté.

15,8 g de phényl thio-4 butyronitrile sont ajoutés goutte à goutte à 20 °C en 2 min à une solution de 18 ml de tribromure de phosphore dans 25 ml de dioxanne. Après 12 h à 20 °C, on ajoute 10 ml d'eau distillée en maintenant la température à 0 °C puis on chauffe à 50–60 °C pendant 3 h. On ajoute ensuite 100 ml d'eau et le précipité formé est filtré, lavé à l'eau, à l'acétone et enfin au méthanol puis séché sous vide. Après séchage, le produit est repris par une solution de 8,5 g de soude dans 100 ml d'eau. Après dissolution complète, on ajoute 200 ml de méthanol au mélange. Le précipité formé est filtré, lavé au méthanol puis séché à 80 °C sous vide. On obtient 17,9 g du produit attendu. F > 300 °C.

Analyse élémentaire avec 3 molécules d'eau

| | | | | | | |
|---|---|---|---|---|---|---|
| Calculé | C% | 24,85 | H% | 3,96 | N% | 2,89 |
| Trouvé | | 24,42 | | 3,92 | | 2,88 |

Exemple 6

Sel tétrasodique de l'acide amino-1 (chloro-4 phényl thio)-4 butylidène diphosphonique-1,1 (SR 42509 A).

$R_1$ = 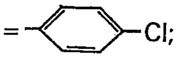 ; n=0; Alk=$(CH_2)_3$; $R_2$=$R_3$=H I

a) Chloro-4 phényl)thio-4 butyronitrile.

A une solution de 4 g de sodium dans 200 ml de méthanol, on ajoute 44 g de chloro-4 thiophénol. Après 5 min de chauffage à reflux, on ajoute doucemente 32 g de chloro-4 butyronitrile. On chauffe ensuite à reflux pendant 3 h puis on refroidit et concentre la solution sous vide. Le résidu est repris par 300 ml d'éther isopropylique. La solution est lavée à l'eau, séchée puis concentrée. Le résidu est distillé et la fraction qui distille entre 150 g et 155 °C sous 0,03 mm de mercure est séparée. On obtient ainsi 53,5 g du nitrile attendu sous forme d'une huile incolore caractérisée par un RF de 0,64 en chromatographie en couche mince sur gel de silice (Kieselgel 60 F 54 Merck) avec l'éther isopropylique comme éluant:

b) Sel tétrasodique de l'acide amino-1 (chloro-4 phényl thio)-4 butylidène diphosphonique-1,1.

21,1 g de (chloro-4 phényl)thio-4 butyronitrile sont ajoutés à une solution de 19,6 ml de tribromure de phosphore dans 30 ml de dioxanne en 5 min, en maintenant la température à 30 °C. Le mélange est ensuite agité 20 h à 30 °C puis 2 h à 45 °C. On ajoute alors 50 ml d'eau en laissant la température monter à 80 °C. Le précipité formé est filtré, lavé au dioxanne puis repris par 100 ml d'eau. Le mélange est chauffé 1 h à reflux et, après refroidissement, l'insoluble est filtré et lavé à l'alcool absolu et enfin repris par une solution de 100 ml de soude à 5%. Après dissolution totale, on ajoute 500 ml de méthanol. Le précipité formé est filtré, lavé au méthanol et séché pour conduire à 12,5 g de produit attendu. F > 300 °C.

Analyse élémentaire

| | | | | |
|---|---|---|---|---|
| Calculé | C% | 25,90 | N% | 3,02 |
| Trouvé | | 26,25 | | 3,10 |

Exemple 7

Sel tétrasodique de l'acide amino-1 cyclohexyl thio-4 butylidène diphosphonique-1,1, monohydraté (SR 42684 A).

$R_1$= 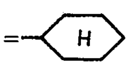 ; n=0; Alk=$(CH_2)_3$; $R_2$=$R_3$=H

I

a) Cyclohexyl thio-4 butyronitrile.

A une solution de 3,5 g de sodium dans 75 ml de méthanol, on ajoute 18 g de cyclohexanethiol. On agite à 20 °C pendant 1 h puis on ajoute 14,2 ml de chloro-4 butyronitrile et le mélange est chauffé à reflux pendant 3 h avant de concentrer sous vide. Le résidu est repris par 300 ml d'éther; la solution est lavée à l'eau, puis séchée et concentrée. Le résidu distillé sous un vide de 0,03 mm de mercure fournit 20,8 g d'une huile incolore qui distille entre 120 et 125 °C. Le cyclohexyl thio-4 butyronitrile ainsi préparé est caractérisé par un RF de 0,70 en chromatographie en couche mince sur silice (Kieselgel 60 F 54 Merck); l'éther isopropylique étant utilisé comme éluant.

b) Sel tétrasodique de l'acide amino-1 cyclohexyl thio-4 butylidène diphosphonique-1,1, monohydraté.

Le mélange de 11 g d'acide phosphoreux et de 10 g de cyclohexyl thio-4 butyronitrile est chauffé à 160 °C pendant 3 h. Le résidu est repris par 40 ml d'eau: il cristallise alors un solide jaune qui est lavé à l'eau puis à l'acétone et enfin à l'éther. Ce composé est repris par une solution de 1,5 g de soude dans 70 ml d'eau distillée. On chauffe le mélange à 50 °C sous agitation jusqu'à dissolution totale: on laisse refroidir et on ajoute 250 ml de méthanol. Le précipité incolore obtenu est filtré, lavé au méthanol puis séché à 80 °C sous 0,1 mm de mercure. F > 300 °C.

Analyse élémentaire avec 1 molécule d'eau

| | | | |
|---|---|---|---|
| Calculé | C% 26,49 | H% 4,67 | N% 3,08 |
| Trouvé | 25,93 | 4,90 | 3,04 |

Exemple 8

Disel de sodium de l'acide amino-1 (pyridyl-2 thio)-5 pentylidène diphosphonique-1,1 (SR 42709 A).

$R_1$ = 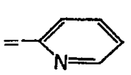 ; n=0; Alk=$(CH_2)_4$; $R_2$=$R_3$=H I

a) (Pyridinyl-2 thio)-5 valéronitrile.

A une solution de 2,26 g de sodium dans 200 ml de méthanol, on ajoute successivement 10 g de mercapto-2 pyridine et 20 g de brome-6 valéronitrile. Après 3 h de chauffage à reflux, on concentre sous vide. Le résidu est repris par 200 ml d'éther. La solution est lavée à l'eau puis séchée et concentrée: on obtient ainsi 15 g du nitrile attendu caractérisé par un RF de 0,39 en chromatographie en couche mince sur silice (Kieselgel 60 F 54 Merck) avec l'éther isopropylique comme éluant.

b) Disel de sodium de l'acide amino-1 (pyridyl-2 thio)-5 pentylidène diphosphonique-1,1.

On chauffe à 150°C pendant 1 h puis à 170°C pendant 3 h le mélange de 10,2 g de (pyridyl-2 thio)-5 valéronitrile et 12 g d'acide phosphoreux. Après refroidissement, le mélange est repris par 100 ml d'eau et on ajoute 2,5 g d'acétate de sodium.

Le milieu est ensuite extrait deux fois à l'éther et la phase aqueuse est séparée; on ajoute 300 ml de méthanol dans cette dernière; le précipité formé est filtré, lavé au méthanol puis à l'éther et enfin recristallisé dans un mélange eau-méthanol (60/40). On obtient ainsi 4,2 g du produit attendu. F > 300 °C.

Analyse élémentaire

| | | | |
|---|---|---|---|
| Calculé | C% 30,00 | H% 4,03 | N% 7,00 |
| Trouvé | 30,00 | 4,20 | 7,02 |

Exemple 9

Disel de sodium de l'acide amino-1 propylthio-6 hexylidène diphosphonique-1,1 (SR 42708 A).

$R_1 = -(CH_2)_2-CH_3$; n=0;

Alk= $-(CH_2)_5-$; $R_2$=$R_3$=H I

a) Cyano-1 propylthio-5 pentane.

A une solution de 8 g de sodium dans 600 ml d'éthanol, on ajoute successivement 26 g de propanethiol puis 45 g de cyano-1 bromo-5 pentane. Après 6 h de chauffage à reflux, on concentre le solvant puis le résidu est repris par 500 ml d'eau et extrait à l'éther. La phase organique concentrée fournit après chromatographie sur colonne de silice (230–240 Mesh) 28 g du nitrile attendu caractérisé par son RF de 0,60 en chromatographie en couche mince sur gel de silice (Kieselgel 60 F 54 Merck) avec l'éther isopropylique comme éluant (RF = 0,10 avec un mélange acétone-hexane 1/9 comme éluant).

b) Disel de sodium de l'acide amino-1 propyl thio-6 hexylidène diphosphonique-1,1.

Une solution de 17,5 g de cyano-1 propyl thio-5 pentane et 20 g d'acide phosphoreux est chauffée à 150 °C pendant 1 h puis à 180 °C pendant 2 h. Après refroidissement, le précipité est repris dans l'acétone, filtré puis séché. On reprend ensuite par une solution de 40 g d'acétate de sodium dans 400 ml d'eau. Le mélange est agité pendant 1 h puis filtré. On ajoute alors 400 ml de méthanol à la solution aqueuse et on filtre le précipité. Après séchage, on obtient 11,5 g du produit attendu. F > 300 °C.

Analyse élémentaire

| | | | |
|---|---|---|---|
| Calculé | C% 28,6 | H% 5,57 | N% 3,7 |
| Trouvé | 28,1 | 5,84 | 3,46 |

Exemple 10

Monosel de sodium de l'acide méthylamino-1 (chloro-4 phénylthio)-4 butylidène diphosphonique-1,1 (SR 43140 A).

$R_1$ = 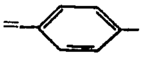 Cl; n=0; Alk=$(CH_2)_3$; $R_2$=$CH_3$;

$R_3$=H I

On chauffe au reflux pendant 3 h le mélange de 20 g de N-méthyl (chloro-4 phénylthio)-4 butyramide, 10 ml de trichlorure de phosphore, 13 g d'acide phosphoreux et 100 ml de diméthoxyéthane.

On laisse ensuite 12 h au repos à température ambiante et on essore les cristaux. On lave avec du méthanol puis avec de l'acétone. Poids: 25 g.

On met l'acide ainsi obtenu en suspension dans 250 ml d'eau puis on ajoute 15 g d'acétate de sodium et on chauffe au reflux pendant 3 h.

Après refroissement, on essore le solide, on lave avec du méthanol puis avec de l'éther et on sèche. Poids 24,25 g; F > 260 °C.

Analyse élémentaire

| | | | |
|---|---|---|---|
| Calculé | C% 32,09 | H% 4,16 | N% 3,40 |
| Trouvé | 32,09 | 3,85 | 3,46 |

Exemple 11 à 22

En opérant comme dans l'exemple 10, mais en faisant varier l'amide de départ, on obtient les produits (I) n=0 réunis dans le tableau 1. Quand l'acide ne cristallise pas par refroidissement du mélange réactionnel, on évapore à siccité sous vide et reprend le résidu dans l'eau.

Pour préparer les sels de sodium, on peut remplacer l'acétate de sodium par de la soude.

Tableau 1

| Ex. n° | N° de code SR | $R_1$ | Alk | $R_2$ | $R_3$ | Acide ou sel Pt fusion °C Analyse: (valeur calculée) |
|---|---|---|---|---|---|---|
| 11 | 43 141 | 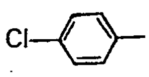 | $(CH_2)_3$ | $-CH_3$ | $-ClH_3$ | Acide F: 202<br>C: 35,70 (35,63)<br>H: 4,99 ( 4,73)<br>N: 3,47 ( 3,43) |
| 12 | 43 162 A | 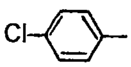 | $(CH_2)_3$ | H | 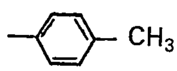 | Sel disodique F > 260 avec 1 $H_2O$<br>C: 38,70 (38,50)<br>H: 3,94 ( 4,19)<br>N: 2,65 ( 2,63) |
| 13 | 43 164 | 〃 | 〃 | H | $-(CH_2)_5CH_3$ | Acide F: 222<br>C: 41,42 (41,79)<br>H: 6,37 ( 6,14)<br>N: 2,99 ( 3,04) |
| 14 | 43 163 | 〃 | 〃 | | $-(CH_2)_5$ | Acide F: 208<br>C: 40,61 (40,59)<br>H: 5,68 ( 5,45)<br>N: 3,11 ( 3,15) |
| 15 | 43 175 A | 〃 | 〃 | H | 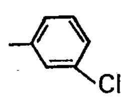 | Sel disodique F > 260 avec 3 $H_2O$<br>C: 32,80 (32,95)<br>H: 3,56 ( 3,94)<br>N: 2,26 ( 2,40) |
| 16 | 43 176 | 〃 | 〃 | H | 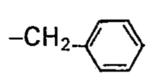 | Acide F: 194 avec 2 $H_2O$<br>C: 41,64 (41,60)<br>H: 4,53 ( 4,82)<br>N: 2,76 ( 2,79) |
| 17 | 43 299 B | 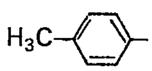 | $CH_2$ | H | 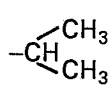 | Sel disodique F > 260 avec 2 $H_2O$<br>C: 32,03 (32,07)<br>H: 5,05 ( 5,15)<br>N: 3,09 ( 3,11) |

Tableau I (suite)

| Ex. n° | N° de code SR | $R_1$ | Alk | $R_2$ | $R_3$ | Acide ou sel Pt fusion °C Analyse: (valeur calculée) |
|---|---|---|---|---|---|---|
| 18 | 43 404 A | $CH_3-CH_2-$ | $(CH_2)_4$ | H | $-CH_3$ | Sel monosodique<br>F > 300<br>C: 28,34 (28,00)<br>H: 5,55 ( 5,87)<br>N: 4,26 ( 4,08) |
| 19 | 43 376 A | 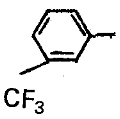 | $(CH_2)_2$ | H | $-CH_3$ | Sel monopotassique<br>F > 300<br>avec 1 $H_2O$<br>C: 28,36 (28,39)<br>H: 3,24 ( 3,68)<br>N: 2,99 ( 3,01) |
| 20 | 43 368 A | 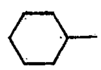 | $(CH_2)_5$ | H | $-CH_3$ | Sel dipotassique<br>F > 300<br>avec 2 $H_2O$<br>C: 31,47 (31,13)<br>H: 5,89 ( 6,22)<br>N: 2,76 ( 2,79) |
| 21 | 43 369 A | 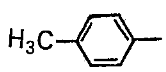 | $-CH-CH_2$<br>\|<br>$CH_3$ | H | $-CH_2CH_2CH_3$ | Sel disodique F: 260<br>avec 1 $H_2O$<br>C: 36,96 (36,61)<br>H: 5,69 ( 5,48)<br>N: 3,07 ( 3,05) |
| 22 | 43 354 A | 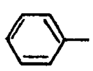 | $(CH_2)_4$ | H | $-CH_3$ | Sel disodique F > 260<br>avec 1/3 $H_2O$<br>C: 31,20 (31,38)<br>H: 4,40 ( 4,45)<br>N: 6,62 ( 6,65) |

Exemple 23

Acide phénylthio-4 uréido-1 butylidène diphosphonique-1,1 (SR 43142)

$R_1=$ 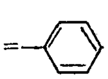 n=0; $R_3=H$; I

$R_2=-C-NH_2$; $Alk=(CH_2)_3$
||
O

On introduit 10 g d'acide amino-1 phénylthio-4 butylidène diphosphonique-1,1 (exemple 5) dans 45 g d'urée en fusion (150 °C) sous agitation et maintient à cette température pendant 90 min. On verse le mélange réactionnel dans 500 ml d'acétone et filtre d'insoluble à chaud. On le traite à nouveau par 1 l d'acétone et essore. On dissout l'insoluble dans l'eau et on le passe sur une colonne de résine sulfonique préalablement activée par H Cl 2N. Après évaporation de l'eau, on obtient un solide qu'on triture avec du chlorure de méthylène. On obtient finalement 5,6 g d'un solide incolore, F: 125–130 °C.

Analyse élémentaire

| | | | | | | |
|---|---|---|---|---|---|---|
| Calculé | C% | 34,38 | H% | 4,72 | N% | 7,29 |
| Trouvé | | 34,10 | | 4,74 | | 7,27 |

Exemple 24

Acide (chloro-4 phénylsulfinyl)-4 méthylamino-1 butylidène diphosphonique-1,1 (SR 43264).

$R_1 =$ 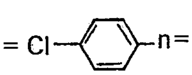 n=1; $Alk = (CH_2)_3$; I

$R_2=H$; $R_3=CH_3$

On chauffe au reflux pendant 3 h le mélange de 8,5 g de N-méthyl (chloro-4 phénylsulfinyl)-4 butyramide, 4 ml de trichlorure de phosphore, 8 g d'acide phosphoreux et 50 ml de diméthoxy-1,2 éthane. On évapore le solvant à siccité sous vide, et reprend le résidu par de l'eau bouillante. On essore le solide, lave avec de l'eau puis avec du méthanol. On obtient 6 g de solide; F > 260 °C.

Analyse élémentaire – Avec $H_2O$

| | | | | | | |
|---|---|---|---|---|---|---|
| Calculé | C% | 31,27 | H% | 4,76 | N% | 3,30 |
| Trouvé | | 31,28 | | 4,78 | | 3,32 |

Exemple 25

Sel monosodique de l'acide méthylamino-1 (méthyl-4 phénylsulfonyl)-4 propylidène diphosphonique-1,1 (SR 43 370 A).

$R_1$ = $H_3C$— 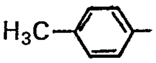 —n=2; Alk=$(CH_2)_2$; $R_2$=H;
$R_3$=$CH_3$ I

On chauffe au reflux pendant 3 h le mélange de 9,5 g de N-méthyl (méthyl-4 phénylsulfonyl)-3 propionamide, 9 ml de trichlorure de phosphore, 6 g d'acide phosphoreux et 50 ml de diméthoxy-1,2 éthane. On concentre à siccité sous vide puis on reprend le résidu dans 200 ml d'eau bouillante. Après refroidissement on filtre la solution et on ajoute 20 g d'acétate de sodium. On laisse 12 h et essore le solide qu'on lave 2 fois avec de l'eau puis avec de l'acétone.

Le sel de sodium brut est mis en suspension dans 100 ml d'eau distillée. On ajoute 1 ml d'acide chlorhydrique concentré et chauffe au reflux pendant 30 min. Après refroidissement, on essore le précipité qu'on lave à l'eau et au méthanol. Après séchage on obtient 3,6 g du produit attendu;F > 300 °C.

Analyse élémentaire

| | | | | | | |
|---|---|---|---|---|---|---|
| Calculé | C% | 32,12 | H% | 4,41 | N% | 3,41 |
| Trouvé | | 31,79 | | 4,21 | | 3,39 |

Les composés selon l'invention sont avantageusement utilisés comme médicaments anti-inflammatoires et antirhumatismaux et leurs propriétés pharmacologiques ont été mises en évidence de la manière suivante:

Etude «in vivo»: arthrite à l'adjuvant

L'injection de mycobacterium chez le rat entraîne une polyarthrite rappelant, par certains aspects, l'arthrite rhumatoïde humaine.

Protocole

Une suspension de Mycobacterium Tuberculosis (0,4 mg) dans l'huile de paraffine (0,05 ml) est injectée par voie intradermique dans la queue de rats mâles Sprague-Dawley d'un poids moyen de 150 g.

Après 15 jours, on trie les animaux présentant les symptômes d'arthrite les plus marqués. Ces rats sont répartis en 2 lots de 14 animaux.

Les animaux du 1er groupe sont traités 6 jours/semaine pendant 3 semaines:

– soit par voie sous-cutanée à des doses allant de 0,32 µmol à 32 µmol/kg/jour,

– soit par voie orale à la dose de 0,16 mmol/kg/jour.

Le second groupe reçoit un placébo et sert de témoin.

Après 3 semaines de traitement, la moitié des animaux de chaque groupe est sacrifiée; l'autre moitié est conservée sans traitement pendant 2 semaines puis sacrifiée à son tour.

Après sacrifice, la patte arrière droite est sectionnée au niveau de l'articulation tibiotarsienne puis pesée. Pour chaque demi-groupe on détermine le moyenne et l'erreur standard des poids.

L'activité du produit est exprimée par la variation en % du poids moyen des pattes de rats arthritiques traités par rapport à celui des pattes arthritiques des rats non traités (témoins).

Les résultats obtenus avec divers produits de l'invention sont réunis dans le tableau 2 ci-après. Pour chacun des produits testés, l'activité thérapeutique est exprimée en pourcentage d'inhibition du poids de la patte après trois semaines de traitement.

Pour quelques produits, on a également mentionné le résultat obtenu après cinq semaines, soit 3 semaines de traitement suivies de 2 semaines sans traitement.

Tableau 2

| Produit Nº SR | Voie d'administration | % d'inhibition du poids de la patte à la fin du traitement (3 semaines) | 2 semaines après la fin du traitement |
|---|---|---|---|
| 42 509 A | Orale | 17 | 22 |
| 42 683 A | Orale | 9 | 13 |
| 42 708 A | Orale | 28 | 22 |
| 42 709 A | Orale | 0 | 14 |
| 42 710 A | Orale | 19 | 7 |
| 42 718 A | Orale | 35 | 16 |
| 43 140 A | sous-cutanée | 52 | |
| 43 141 | sous-cutanée | 36 | |
| 43 142 | sous-cutanée | 37 | |
| 43 162 A | sous-cutanée | 49 | |

Tableau 2 (suite)

| Produit N° SR | Voie d'administration | % d'inhibition du poids de la patte à la fin du traitement (3 semaines) | 2 semaines après la fin du traitement |
|---|---|---|---|
| 43 163 | sous-cutanée | 35 | |
| 43 164 | sous-cutanée | 35 | |
| 43 175 A | sous-cutanée | 22 | |
| 43 176 | sous-cutanée | 52 | |
| 43 264 | sous-cutanée | 29 | |
| 43 299 B | sous-cutanée | 25 | |
| 43 304 A | sous-cutanée | 30 | |
| 43 354 A | sous-cutanée | 44 | |
| 43 368 A | sous-cutanée | 20 | |
| 43 369 A | sous-cutanée | 38 | |
| 43 370 A | sous-cutanée | 32 | |
| 43 376 A | sous-cutanée | 38 | |

Ces résultats montrent une activité antirhumatismale importante des produits selon l'invention après un traitement de 3 semaines.

Il faut noter que cette activité se maintient à un niveau élevé et quelquefois s'accroît même 2 semaines après la fin du traitement.

Par ailleurs, les produits selon l'invention sont peu toxiques.

Ils peuvent être utilisés en thérapeutique humaine pour le traitement des affections dues à des phénomènes inflammatoires et en particulier pour le traitement des états arthritiques. Notamment, les composés selon l'invention peuvent être utilisés dans le traitement de la polyarthrite rhumatoïde.

L'invention concerne également les compositions pharmaceutiques contenant, à titre d'ingrédient actif, un dérivé selon l'invention en association avec un véhicule pharmaceutiquement acceptable.

Les compositions selon l'invention peuvent être présentées sous les formes appropriées à l'administration par les voies orale, endorectale et parentérale.

Il peut s'agir notamment de gélules ou de comprimés contenant une quantité de principe actif de 10 à 500 mg/unité.

La posologie quotidienne de ces produits chez l'adulte peut être de l'ordre de 100 mg à 5 g par jour répartis en plusieurs prises.

A titre d'exemple, on peut donner la composition galénique suivante:

Gélules

| | |
|---|---|
| SR 42 708A | 200 mg |
| Aérosil | 1 mg |
| Stéarate de magnésium | 3 mg |
| Amidon STARX 1500 | 96 mg |
| | 300 mg. |

**Revendications pour les Etats contractants BE, CH, DE, FR, GB, IT, LI, LU, NL, SE**

1. Dérivés de l'acide méthylènediphosphonique de formule:

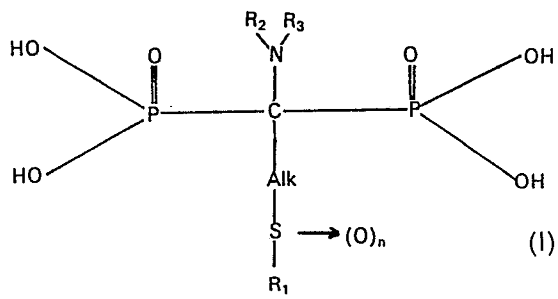

(I)

dans laquelle:

$R_1$ représente:

– un groupe alkyle en $C_1$–$C_6$

– un groupe cycloalkyle en $C_5$–$C_7$

– un groupe phényle éventuellement substitué une ou plusieurs fois par un halogène, un groupe alkyle en $C_1$–$C_6$, un groupe trifluorométhyle,

– un hétérocycle à 5 ou 6 chaînons comportant 1 ou 2 hétéroatomes choisis parmi l'azote et le soufre,

Alk désigne un groupe alkylène droit ou ramifié en $C_1$–$C_6$,

$R_2$ représente l'hydrogène, un groupe alkyle en $C_1$–$C_6$ ou un groupe $-CONH_2$;

$R_3$ représente l'hydrogène, un groupe alkyle en $C_1$–$C_6$, un groupe benzyle ou un groupe phényle éventuellement substitué par un chlore ou un méthyle;

ou encore $R_2$ et $R_3$ pris ensemble représentent un groupe $(CH_2)_m$ où m = 4 ou 5 enfin, n représente un nombre entier 0, 1 ou 2, à la condition que $R_1$ soit différent de $CH_3$ lorsque Alk est $-CH_2-CH_2-$, $R_2=R_3=H$ et n = 0, et les sels desdits dérivés avec des bases minérales ou organiques.

2. Procédé pour l'obtention des dérivés de formule (I) selon la revendication 1, dans laquelle $R_2$ et $R_3$ sont l'hydrogène, caractérisé en ce qu'il consiste à chauffer un nitrile de formule $R_1-\underset{(O)_n}{\underset{|}{S}}-Alk-CN$, dans laquelle $R_1$, Alk et n sont tels que définis dans la revendication 1, en présence d'acide phosphoreux à une température comprise entre 140 °C et 200 °C et éventuellement à transformer l'acide obtenu en l'un de ses sels.

3. Procédé pour l'obtention des dérivés de formule (I) selon la revendication 1, dans laquelle $R_2$ et $R_3$ sont l'hydrogène, caractérisé en ce qu'il consiste à ajouter un nitrile de formule $R_1-\underset{(O)_n}{\underset{|}{S}}-Alk-CN$ dans laquelle $R_1$, Alk et n sont tels que définis dans la revendication 1, à une solution de tribromure de phosphore dans un solvant convenable en présence d'eau, à agiter le mélange résultant à une température entre 30 et 70 °C et éventuellement à transformer l'acide ainsi obtenu en l'un de ses sels.

4. Procédé pour l'obtention des dérivés selon la revendication 1, caractérisé en ce qu'il consiste à chauffer l'amide de formule:

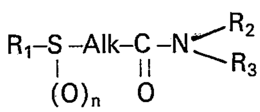

dans laquelle $R_1$, $R_2$, $R_3$, Alk et n sont tels que définis dans la revendication 1, avec de l'acide phosphoreux et du trichlorure de phosphore au sein d'un solvant inerte à une température comprise entre 50 et 100 °C, et éventuellement à transformer l'acide obtenu en l'un de ses sels.

5. Composition pharmaceutique à action antirhumatismale, caractérisée en ce qu'elle contient, à titre d'ingrédient actif, un dérivé de l'acide méthylènediphosphonique de formule:

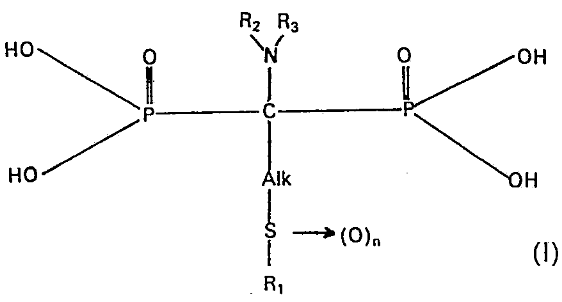

(I)

dans laquelle:

$R_1$ représente:

– un groupe alkyle en $C_1$–$C_6$

– un groupe cycloalkyle en $C_5$–$C_7$

– un groupe phényle éventuellement substitué une ou plusieurs fois par un halogène, un groupe alkyle en $C_1$–$C_6$, un groupe trifluorométhyle,

– un hétérocycle à 5 ou 6 chaînons ccmportant 1 ou 2 hétéroatomes choisis parmi l'azote et le soufre,

Alk désigne un groupe alkylène droit ou ramifié en $C_1$–$C_6$,

$R_2$ représente l'hydrogène, un groupe alkyle en $C_1$–$C_6$ ou un groupe $-CONH_2$;

$R_3$ représente l'hydrogène, un groupe alkyle en $C_1$–$C_6$, un groupe benzyle ou un groupe phényle éventuellement substitué par un chlore ou un méthyle;

ou encore $R_2$ et $R_3$ pris ensemble représentent un groupe $(CH_2)_m$ où m=4 ou 5 enfin, n représente un nombre entier 0, 1 ou 2, ou un des sels dudit dérivé avec des bases organiques ou minérales, en association avec un véhicule pharmaceutique acceptable.

6. Composition pharmaceutique à action antirhumatismale, caractérisée en ce qu'elle cintient, à titre d'ingrédient actif, un dérivé de l'acide méthylènediphosphonique de formule:

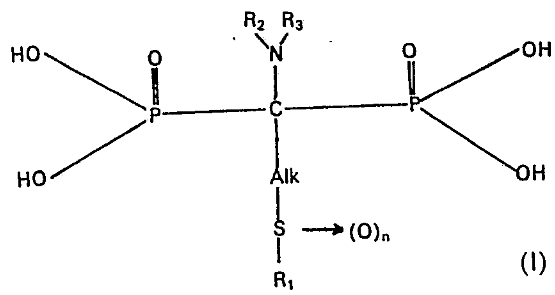

(I)

dans laquelle $R_1$, $R_2$, $R_3$, Alk et n sont tels que définis à la revendication 5, ou un des sels dudit dérivé avec des bases organiques ou minérales, en association avec un véhicule pharmaceutiquement acceptable.

7. Composition selon la revendication 6, carastérisée en ce qu'elle contient 10 à 500 mg dudit dérivé.

**Revendications pour l'Etat contractant AT**

1. Procédé pour l'obtention de dérivés de l'acide méthylène-diphosphonique répondant à la formule:

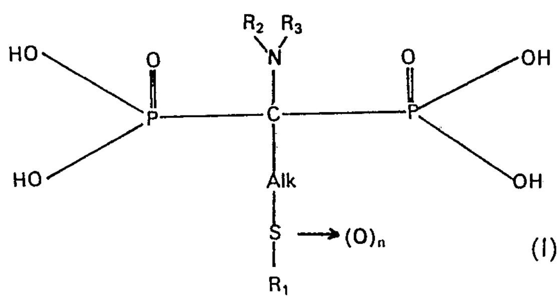

(I)

dans laquelle:

$R_1$ représente:

– un groupe alkyle en $C_1$–$C_6$

– un groupe cycloalkyle en $C_5$–$C_7$

– un groupe phényle éventuellement substitué une ou plusieurs fois par un halogène, un groupe alkyle en $C_1$–$C_6$, un groupe trifluorométhyle,

– un hétérocycle à 5 ou 6 chaînons comportant 1 ou 2 hétéroatomes choisis parmi l'azote et le soufre,

Alk désigne un groupe alkylène droit ou ramifié en $C_1$–$C_6$,

$R_2$ représente l'hydrogène, un groupe alkyle en $C_1$–$C_6$ ou un groupe $-CONH_2$;

$R_3$ représente l'hydrogène, un groupe alkyle en $C_1$–$C_6$, un groupe benzyle ou un groupe phényle éventuellement substitué par un chlore ou un méthyle;

ou encore $R_2$ et $R_3$ pris ensemble représentent un groupe $(CH_2)_m$ où m=4 ou 5 enfin, n représente un nombre entier 0, 1 ou 2, à la condition que $R_1$ soit différent de $CH_3$ lorsque Alk est $-CH_2-CH_2-$, $R_2=R_3=H$ et n=0, et les sels desdits dérivés avec des bases minérales ou organiques, caractérisé en ce qu'il consiste à chauffer l'amide de formule:

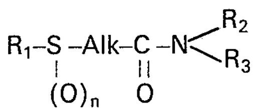

dans laquelle $R_1$, $R_2$, $R_3$, Alk et n sont tels que définis ci-dessus avec de l'acide phosphoreux et du trichlorure de phosphore au sein d'un solvant inerte à une température comprise entre 50 et 100 °C et éventuellement à transformer l'acide ainsi obtenu en l'un de ses sels.

2. Procédé pour l'obtention de dérivés de l'acide méthylènediphosphonique de formule (I) selon la revendication 1, dans laquelle $R_2$ et $R_3$ sont l'hydrogène, caractérisé en ce qu'il consiste à chauffer un nitrile de formule:

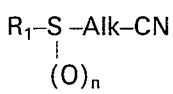

dans laquelle $R_1$, Alk et n sont tels que définis cidessus en présence d'acide phosphoreux à une température comprise entre 140 et 200 °C et éventuellement à transformer l'acide obtenu en l'un de ses sels.

3. Procédé pour l'obtention de dérivés de l'acide méthylènediphosphonique de formule (I) selon la revendication 1, dans laquelle $R_2$ et $R_3$ sont l'hydrogène, caractérisé en ce qu'il consiste à ajouter un nitrile de formule:

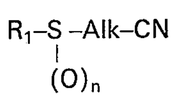

dans laquelle $R_1$, Alk et n sont tels que définis dans la revendication 1, à une solution de tribromure de phosphore dans un solvant convenable en présence d'eau, à agiter le mélange résultant à une température entre 30 et 70 °C et éventuellement à transformer l'acide ainsi obtenu en l'un de ses sels.

4. Utilisation d'un dérivé de l'acide méthylène diphosphonique de formule:

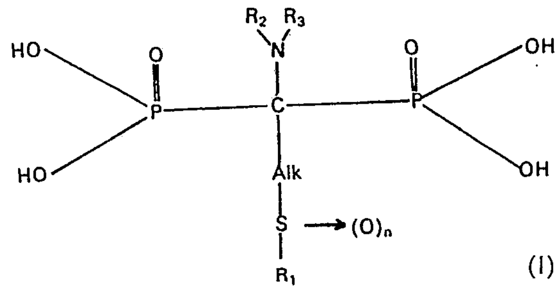

(I)

dans laquelle:

$R_1$ représente:

– un groupe alkyle en $C_1$-$C_6$

– un groupe cycloalkyle en $C_5$-$C_7$

– un groupe phényle éventuellement substitué une ou plusieurs fois par un halogène, un groupe alkyle en $C_1$-$C_6$, un groupe trifluorométhyle,

– un hétérocycle à 5 ou 6 chaînons comportant 1 ou 2 hétéroatomes choisis parmi l'azote et le soufre,

Alk désigne un groupe alkylène droit ou ramifié en $C_1$-$C_6$,

$R_2$ représente l'hydrogène, un groupe alkyle en $C_1$-$C_6$ ou un groupe $-CONH_2$;

$R_3$ représente l'hydrogène, un groupe alkyle en $C_1$-$C_6$, un groupe benzyle ou un groupe phényle éventuellement substitué par un chlore ou un méthyle;

ou encore $R_2$ et $R_3$ pris ensemble représentent un groupe $(CH_2)_m$ ou m = 4 ou 5 enfin, n représente un nombre entier 0, 1 ou 2, ou de l'un de ses sels, avec des bases organiques ou minérales, en association avec un véhicule pharmaceutiquement acceptable, pour la préparation de compositions pharmaceutiques.

5. Utilisation d'un dérivé de l'acide méthylènediphosphonique de formule:

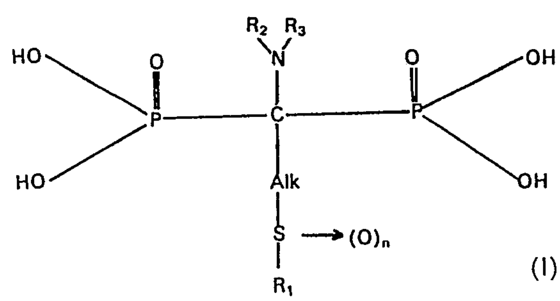

(I)

dans laquelle $R_1$, $R_2$, $R_3$, Alk et sont tels que définis à la revendication 4, ou de l'un de ses sels avec des bases organiques ou minérales, en association avec un véhicule pharmaceutiquement acceptable, pour la préparation de compositions pharmaceutiques ayant une action antirhumatismale.

Utilisation selon la revendication 5, pour la préparation de compositions pharmaceutiques contenant de 10 à 500 mg dudit dérivé ou de son sel.

**Patentansprüche für die Vertragsstaaten BE, CH, DE, FR, GB, IT, LI, LU, NL, SE:**

1. Methylendiphosphonsäure-Derivate der Formel

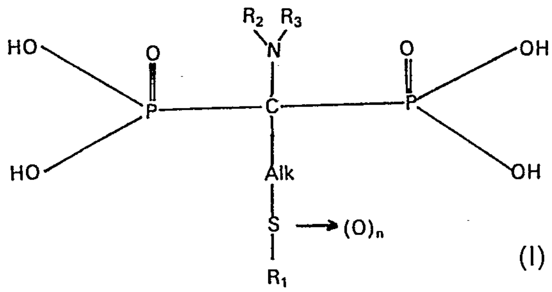

(I)

worin:

$R_1$ darstellt:

– eine $C_1$–$C_6$-Alkylgruppe

– eine $C_5$–$C_7$-Cycloalkylgruppe

– eine gegebenenfalls ein- oder mehrmals durch ein Halogen substituierte Phenylgruppe, eine $C_1$–$C_6$-Alkylgruppe, eine Trifluormethylgruppe,

– einen Heterozyklus mit 5 bis 6 Ketten, umfassend 1 oder 2 Heteroatome, ausgewählt aus Stickstoff und Schwefel;

Alk für eine gerade oder verzweigte $C_1$–$C_6$-Alkylengruppe steht;

$R_2$ Wasserstoff, eine $C_1$–$C_6$-Alkylgruppe oder eine Gruppe $-CONH_2$ darstellt;

$R_3$ Wasserstoff, eine $C_1$–$C_6$-Alkylgruppe, eine Benzylgruppe oder eine gegebenenfalls durch ein Chlor oder ein Methyl substituierte Phenylgruppe bezeichnet;

oder aber $R_2$ und $R_3$ zusammen genommen eine Gruppe $(CH_2)_m$, worin m=4 oder 5, darstellen;

und schliesslich n für eine ganze Zahl 0, 1 oder 2 steht, mit der Massgabe, dass $R_1$ nicht $CH_3$ ist, wenn Alk für $-CH_2-CH_2-$ steht, $R_2=R_3=H$ und n=0, und die Salze der Derivate mit Mineral- oder organischen Basen.

2. Verfahren zur Herstellung der Derivate der Formel (I) nach Anspruch 1, worin $R_2$ und $R_3$ Wasserstoff sind, dadurch gekennzeichnet, dass es darin besteht, dass ein Nitril der Formel

$R_1S$–Alk–CN, worin $R_1$, Alk und n wie in An-
|
$(O)_n$

spruch 1 definiert sind, in Gegenwart von phosphoriger Säure auf eine Temperatur zwischen 140 °C und 200 °C erhitzt und die erhaltene Säure gegebenenfalls in eines ihrer Salze übergeführt wird.

3. Verfahren zur Herstellung der Derivate der Formel (I) nach Anspruch 1, worin $R_2$ und $R_3$ Wasserstoff sind, dadurch gekennzeichnet, dass es darin besteht, dass ein Nitril der Formel

$R_1$–S–Alk–CN, worin $R_1$, Alk und n wie in An-
|
$(O)_n$

spruch 1 definiert sind, zu einer Phosphortribromidlösung in einem geeigneten Lösungsmittel in Gegenwart von Wasser gegeben wird, die resultierende Mischung bei einer Temperatur zwischen 30 und 70 °C gerührt und gegebenenfalls die so erhaltene Säure in eines ihrer Salze übergeführt wird.

4. Verfahren zur Herstellung der Derivate nach Anspruch 1, dadurch gekennzeichnet, dass es darin besteht, dass das Amid der Formel

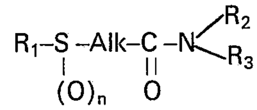

worin $R_1$, $R_2$, $R_3$, Alk und n wie in Anspruch 1 definiert sind, mit phosphoriger Säure und Phosphortrichlorid in einem inerten Lösungsmittel auf eine Temperatur zwischen 50 und 100 °C erhitzt und die erhaltene Säure gegebenenfalls in eines ihrer Salze übergeführt wird.

5. Pharmazeutische Zusammensetzung, dadurch gekennzeichnet, dass sie als aktives Ingrediens ein Methylendiphosphonsäure-Derivat der Formel

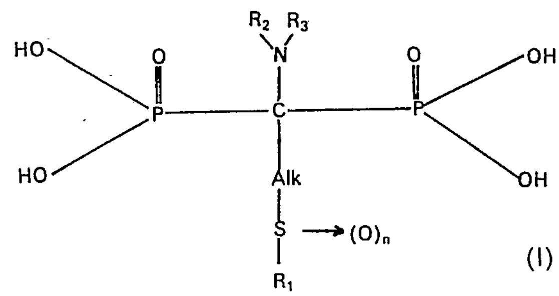

(I)

worin:

$R_1$ darstellt:

– eine $C_1$–$C_6$-Alkylgruppe

– eine $C_5$–$C_7$-Cycloalkylgruppe

– eine gegebenenfalls ein- oder mehrmal durch ein Halogen substituierte Phenylgruppe, eine $C_1$–$C_6$-Alkylgruppe, eine Trifluormethylgruppe,

– einen Heterozyklus mit 5 bis 6 Ketten, umfassend 1 oder 2 Heteroatome, ausgewählt aus Stickstoff und Schwefel;

Alk für eine gerade oder verzweigte $C_1$–$C_6$-Alkylengruppe steht;

$R_2$ Wasserstoff, eine $C_1$–$C_6$-Alkylgruppe oder eine Gruppe $-CONH_2$ darstellt;

$R_3$ Wasserstoff, eine $C_1$–$C_6$-Alkylgruppe, eine Benzylgruppe oder eine gegebenenfalls durch ein Chlor oder ein Methyl substituierte Phenylgruppe bezeichnet;

oder aber $R_2$ und $R_3$ zusammen genommen eine Gruppe $(CH_2)_m$, worin m=4 oder 5, darstellen;

und schliesslich n für eine ganze Zahl 0, 1 oder 2 steht; oder eines der Salze des Derivats von organischen oder Mineralbasen in Verbindung mit einem akzeptablen pharmazeutischen Vehikel enthält.

6. Pharmazeutische Zusammensetzung mit antirheumatischer Wirkung, dadurch gekennzeichnet, dass sie als aktives Ingrediens ein Methylendiphosphonsäure-Derivat der Formel

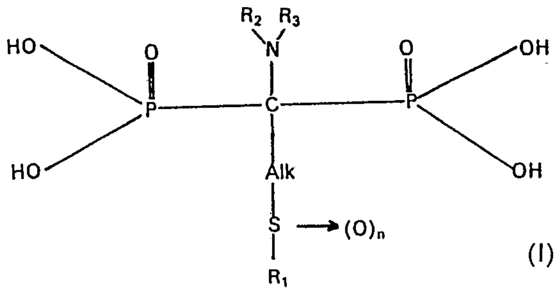

(I)

worin $R_1$, $R_2$, $R_3$, Alk und n wie in Anspruch 5 definiert sind, oder eines der Salze des Derivats mit organischen oder Mineralbasen in Verbindung mit einem pharmazeutisch akzeptablen Vehikel enthält.

7. Zusammensetzung nach Anspruch 6, dadurch gekennzeichnet, dass sie 10 bis 500 mg des Derivats enthält.

**Patentansprüche für den Vertragsstaat AT:**

1. Verfahren zur Herstellung von Methylendiphosphonsäure-Derivaten der Formel

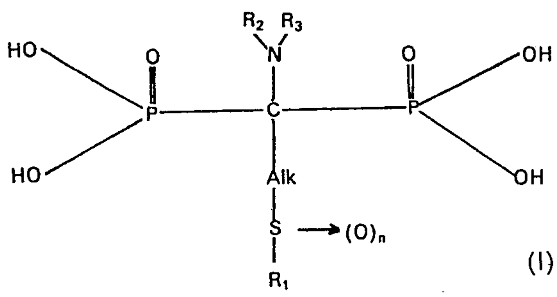

(I)

worin:

$R_1$ darstellt:

- eine $C_1$–$C_6$-Alkylgruppe
- eine $C_5$–$C_7$-Cycloalkylgruppe
- eine gegebenenfalls ein- oder mehrmal durch ein Halogen substituierte Phenylgruppe, eine $C_1$–$C_6$-Alkylgruppe, eine Trifluormethylgruppe,
- einen Heterozyklus mit 5 bis 6 Ketten, umfassend 1 oder 2 Heteroatome, ausgewählt aus Stickstoff und Schwefel;

Alk für eine gerade oder verzweigte $C_1$–$C_6$-Alkylengruppe steht;

$R_2$ Wasserstoff, eine $C_1$–$C_6$- Alkylgruppe oder eine Gruppe $-CONH_2$ darstellt;

$R_3$ Wasserstoff, eine $C_1$–$C_6$-Alkylgruppe, eine Benzylgruppe oder eine gegebenenfalls durch ein Chlor oder ein Methyl substituierte Phenylgruppe bezeichnet;

oder aber $R_2$ und $R_3$ zusammen genommen eine Gruppe $(CH_2)_m$, worin m=4 oder 5, darstellen;

und schliesslich n für eine ganze Zahl 0, 1 oder 2 steht, mit der Massgabe, dass $R_1$ nicht $CH_3$ ist, wenn Alk für $-CH_2-CH_2-$ steht, $R_2=R_3=H$ und n=0,

und der Salze der Derivate mit Mineral- oder organischen Basen, dadurch gekennzeichnet, dass es darin besteht, dass das Amid der Formel

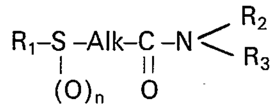

worin $R_1$, $R_2$, $R_3$, Alk und wie oben definiert sind, mit phosphoriger Säure und Phosphortrichlorid in einem inerten Lösungsmittel auf eine Temperatur zwischen 50 und 100 °C erhitzt und die erhaltene Säure gegebenenfalls in eines ihrer Salze übergeführt wird.

2. Verfahren zur Herstellung von Methylendiphosphonsäure-Derivaten der Formel (I) nach Anspruch 1, worin $R_2$ und $R_3$ Wasserstoff sind, dadurch gekennzeichnet, dass es darin besteht, dass ein Nitril der Formel

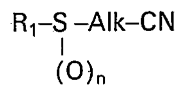

worin $R_1$, Alk und n wie oben definiert sind, in Gegenwart von phosphoriger Säure auf eine Temperatur zwischen 140 °C und 200 °C erhitzt und die erhaltene Säure gegebenenfalls in eines ihrer Salze übergeführt wird.

3. Verfahren zur Herstellung von Methylendiphosphonsäure-Derivaten der Formel (I) nach Anspruch 1, worin $R_2$ und $R_3$ Wasserstoff sind, dadurch gekennzeichnet, dass es darin besteht, dass ein Nitril der Formel

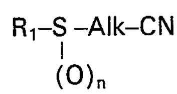

worin $R_1$, Alk und n wie in Anspruch 1 definiert sind, zu einer Phosphortribromidlösung in einem geeigneten Lösungsmittel in Gegenwart von Wasser gegeben wird, die resultierende Mischung bei einer Temperatur zwischen 30 und 70 °C gerührt und gegebenenfalls die so erhaltene Säure in eines ihrer Salze übergeführt wird.

4. Verwendung eines Methylendiphosphonsäure-Derivats der Formel

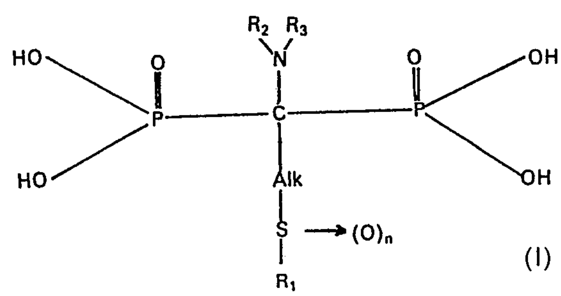

(I)

worin:

$R_1$ darstellt:

- eine $C_1$–$C_6$-Alkylgruppe
- eine $C_5$–$C_7$-Cycloalkylgruppe
- eine gegebenenfalls ein- oder mehrmal durch ein Halogen substituierte Phenylgruppe, eine $C_1$–$C_6$-Alkylgruppe, eine Trifluormethylgruppe,
- einen Heterozyklus mit 5 bis 6 Ketten, umfassend 1 oder 2 Heteroatome, ausgewählt aus Stickstoff und Schwefel;

Alk für eine gerade oder verzweigte $C_1$–$C_6$-Alkylengruppe steht;

$R_2$ Wasserstoff, eine $C_1$–$C_6$-Alkylgruppe oder eine Gruppe –$CONH_2$ darstellt;

$R_3$ Wasserstoff, eine $C_1$–$C_6$-Alkylgruppe, eine Benzylgruppe oder eine gegebenenfalls durch ein Chlor oder ein Methyl substituierte Phenylgruppe bezeichnet;

oder aber $R_2$ und $R_3$ zusammen genommen eine Gruppe $(CH_2)_m$, worin m=4 oder 5, darstellen;

und schliesslich n für eine ganze Zahl 0, 1 oder 2 steht; oder eines seiner Salze mit organischen oder Mineralbasen in Verbindung mit einem pharmazeutisch akzeptablen Vehikel zur Herstellung von pharmazeutischen Zusammensetzungen.

5. Verwendung eines Methylendiphosphonsäure-Derivats der Formel

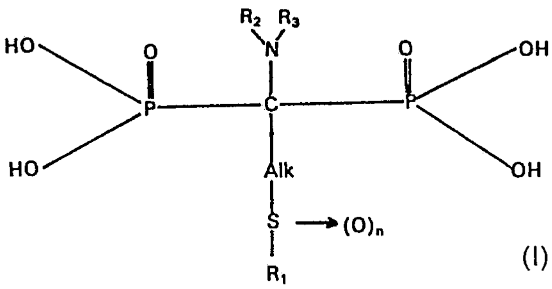

(I)

worin $R_1$, $R_2$, $R_3$, Alk und n wie in Anspruch 4 definiert sind, oder eines der Salze des Derivats mit organischen oder Mineralbasen in Verbindung mit einem pharmazeutisch akzeptablen Vehikel zur Herstellung von pharmazeutischen Zusammensetzungen mit antirheumatischer Wirkung.

6. Verwendung nach Anspruch 5 zur Herstellung von pharmazeutischen Zusammensetzungen enthaltend 10 bis 500 mg des Derivats oder seines Salzes.

**Claims for the contracting states: BE, CH, DE, FR, GB, IT, LI, LU, NL, SE**

1. Methylenediphosphonic acid derivatives of the formula:

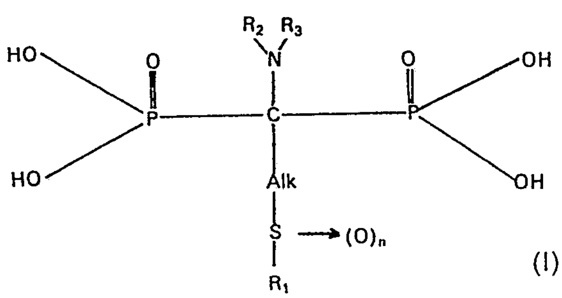

(I)

in which:

$R_1$ represents:

– a $C_1$–$C_6$ alkyl group,

– a $C_5$–$C_7$ cycloalkyl group,

– a phenyl group optionally monosubstituted or polysubstituted by a halogen, a $C_1$–$C_6$ alkyl group or a trifluoromethyl group, or

– a 5-membered or 6-membered heterocycle containing 1 or 2 heteroatoms chosen from nitrogen and sulfur,

Alk denotes a linear or branched $C_1$–$C_6$ alkylene group,

$R_2$ represents hydrogen, a $C_1$–$C_6$ alkyl group or a –$CONH_2$ group,

$R_3$ represents hydrogen, a $C_1$–$C_6$ alkyl group, a benzyl group or a phenyl group optionally substituted by a chlorine or a methyl group;

or alternatively $R_2$ and $R_3$, taken together, represent a $(CH_2)_m$ group, in which m=4 or 5, and finally n represents 0 or the integer 1 or 2, with the proviso that $R_1$ is different from $CH_3$ if Alk is $-CH_2-CH_2-$, $R_2=R_3=H$ and n =0, and the salts of the said derivatives with inorganic or organic bases.

2. A process for the preparation of the derivatives of the formula (I) according to claim 1, in which $R_2$ and $R_3$ are hydrogen, characterized in that it consists in heating a nitrile of the formula $R_1S$–Alk–CN (with $(O)_n$ on S), in which $R_1$, Alk and n are as defined in claim 1, in the presence of phosphorous acid, at a temperature of between 140 °C and 200 °C, and, if appropriate, in converting the resulting acid to one of its salts.

3. A process for the preparation of the derivatives of the formula (I) according to claim 1, in which $R_2$ and $R_3$ are hydrogen, characterized in that it consists in adding a nitrile of the formula $R_1$–S–Alk–CN (with $(O)_n$ on S), in which $R_1$, Alk and n are as defined in claim 1, to a solution of phosphorus tribromide in a suitable solvent, in the presence of water, in stirring the resulting mixture at a temperature of between 30 and 70 °C and, if appropriate, in converting the resulting acid to one of its salts.

4. A process for the preparation of the derivatives according to claim 1, characterized in that it consists in heating the amide of the formula:

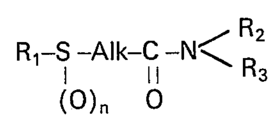

in which $R_1$, $R_2$, $R_3$, Alk and n are as defined in claim 1, with phosphorous acid and phosphorus trichloride, in an inert solvent, at a temperature of between 50 and 100 °C, and, if appropriate, in converting the resulting acid to one of its salts.

5. A pharmaceutical composition, characterized in that it contains, as the active ingredient, a methylenediphosphonic acid derivative of the formula:

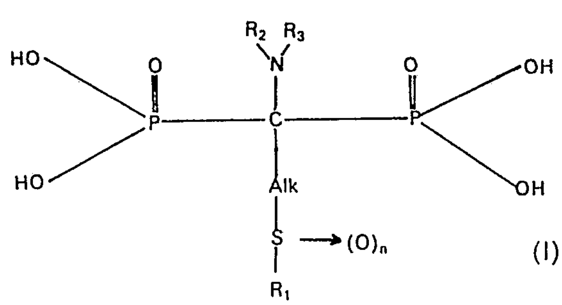

(I)

in which:

$R_1$ represents:

– a $C_1$–$C_6$ alkyl group,

– a $C_5$–$C_7$ cycloalkyl group,

– a phenyl group optionally monosubstituted or polysubstituted by a halogen, a $C_1$–$C_6$ alkyl group or a trifluoromethyl group, or

– a 5-membered or 6-membered heterocycle containing 1 or 2 heteroatoms chosen from nitrogen and sulfur,

Alk denotes a linear or branched $C_1$–$C_6$ alkylene group,

$R_2$ represents hydrogen, a $C_1$–$C_6$ alkyl group or a $-CONH_2$ group,

$R_3$ represents hydrogen, a $C_1$–$C_6$ alkyl group, a benzyl group or a phenyl group optionally substituted by a chlorine or a methyl group;

or alternatively $R_2$ and $R_3$, taken together, represent a $(CH_2)_m$ group, in which m 4 or 5, and finally n represents 0 or the integer 1 or 2,or one of the salts of the said derivative with organic or inorganic bases, in association with a pharmaceutically acceptable vehicle.

6. A pharmaceutical composition having an anti-rheumatic action, characterized in that it contains, as the active ingredient, a methylenediphosphonic acid derivative of the formula:

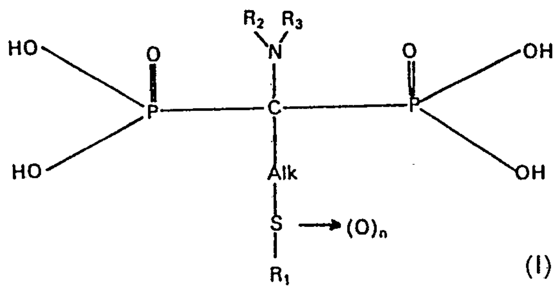

(I)

in which $R_1$, $R_2$, $R_3$ and Alk and n are such as defined in claim 5, or one of the salts of said derivative with organic or inorganic bases, in association with a pharmaceutically acceptable vehicle.

7. A composition according to claim 6, characterized in that it contains 10 to 500 mg of the said derivative.

**Claims for the contracting state: AT**

1. Process for the preparation of methylenediphosphonic acid derivatives of the formula:

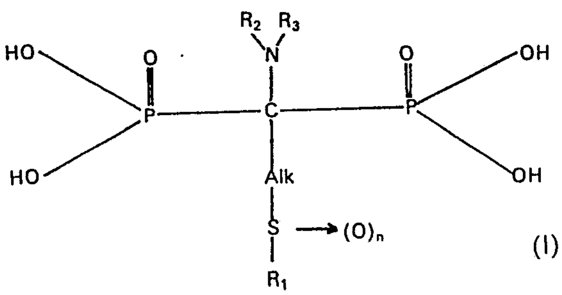

(I)

in which:

$R_1$ represents:

– a $C_1$–$C_6$ alkyl group,

– a $C_5$–$C_7$ cycloalkyl group,

– a phenyl group optionally monosubstituted or polysubstituted by a halogen, a $C_1$–$C_6$ alkyl group or a trifluoromethyl group, or

– a 5-membered or 6-membered heterocycle containing 1 or 2 heteroatoms chosen from nitrogen and sulfur,

Alk denotes a linear or branched $C_1$–$C_6$ alkylene group,

$R_2$ represents hydrogen, a $C_1$–$C_6$ alkyl group or a $-CONH_2$ group,

$R_3$ represents hydrogen, a $C_1$–$C_6$ alkyl group, a benzyl group or a phenyl group optionally substituted by a chlorine or a methyl group;

or alternatively $R_2$ and $R_3$, taken together, represent a $(CH_2)_m$ group, in which m=4 or 5, and finally n represents 0 or the integer 1 or 2, with the proviso that $R_1$ is different from $CH_3$ if Alk is $-CH_2-CH_2-$, $R_2=R_3=H$ and n=0, and the salts of the said derivatives with inorganic or organic bases, characterized in that it consists in heating the amide of the formula:

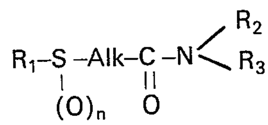

in which $R_1$, $R_2$, $R_3$ and Alk and n are such as defined above with phosphorous acid and phosphorus trichloride, in an inert solvent, at a temperature of between 50 and 100 °C and if appropriate, in converting the resulting acid to one of its salts.

2. A process for the preparation of methylenedisphosphonic acid derivatives of the formula (I) according to claim 1, in which $R_2$ and $R_3$ are hydrogen, characterized in that it consists in heating a nitrile of the formula $R_1S$ –Alk–CN, , in which
(O)$_n$
$R_1$, Alk and n are as defined above, in the presence of phosphorous acid, at a temperature of between 140 °C and 200 °C, and, if appropriate, in converting the resulting acid to one of its salts.

3. A process for the preparaticn of methylenediphosphonic acid derivatives of the formula (I) according to claim 1, in which $R_2$ and $R_3$ are hydrogen, characterized in that it consists in adding a nitrile of the formula $R_1$–S –Alk–CN, in which $R_1$,
(O)$_n$
Alk and n are as defined in claim 1, to a solution of phosphorus tribromide in a suitable solvent, in the presence of water, in stirring the resulting mixture at a temperature of between 30 and 70 °C and, if appropriate, in converting the resulting acid to one of its salts.

4. Use of a methylenediphosphonic acid derivative of the formula:

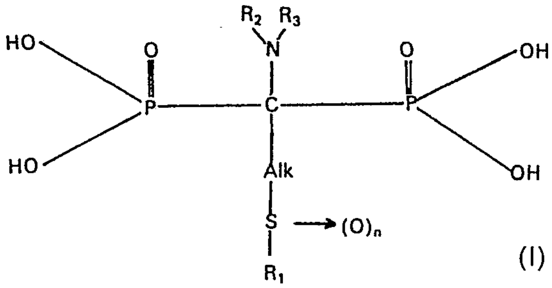

(I)

in which:

$R_1$ represents:

– a $C_1$–$C_6$ alkyl group,

– a $C_5$–$C_7$ cycloalkyl group,

– a phenyl group optionally monosubstituted or polysubstituted by a halogen, a $C_1$–$C_6$ alkyl group or a trifluoromethyl group, or

– a 5-membered or 6-membered heterocycle containing 1 or 2 heteroatoms chosen from nitrogen and sulfur,

Alk denotes a linear or branched $C_1$–$C_6$ alkylene group,

$R_2$ represents hydrogen, a $C_1$–$C_6$ alkyl group or a $-CONH_2$ group,

$R_3$ represents hydrogen, a $C_1$–$C_6$ alkyl group, a benzyl group or a phenyl group optionally substituted by a chlorine or a methyl group;

or alternatively $R_2$ and $R_3$, taken together, represent a $(CH_2)_m$ group, in which m=4 or 5, and finally n represents 0 or the integer 1 or 2, or one of the salts of the said derivative with organic or inorganic bases, in association with a pharmaceutically acceptable vehicle, for the preparation of pharmaceutical compositions.

5. Use of a methylenediphosphonic acid derivative of the formula:

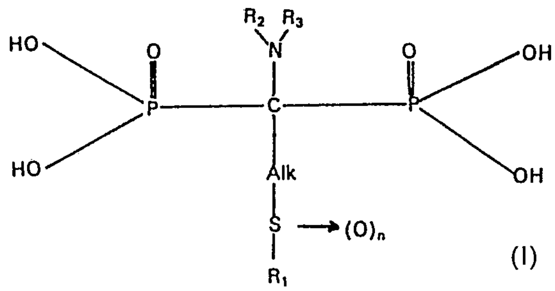

(I)

in which $R_1$, $R_2$, $R_3$, Alk and n are as defined in claim 4, or one of the salts thereof with organic or inorganic bases, in association with a pharmaceutically acceptable vehicle, for the preparation of pharmaceutical compositions having an antirheumatic action.

6. Use according to claim 5, for the preparation of pharmaceutica al compositions containing 10 to 500 mg of the said derivative, or the salt thereof.